# EUROPEAN PATENT SPECIFICATION

(11) **EP 3 753 765 B1**
(45) Date of publication and mention of the grant of the patent: **07.06.2023**
(21) Application number: 19751729.5
(22) Date of filing: 01.02.2019
(51) Int. Cl.: B60H 3/00, A61L 9/12

(54) **INTEGRATED IN-VEHICLE FRAGRANT ASSEMBLY**
INTEGRIERTE FAHRZEUGINTERNE DUFTANORDNUNG
ENSEMBLE PARFUMÉ INTÉGRÉ DANS UN VÉHICULE

(30) Priority: 12.02.2018 CN 201810147009
(43) Date of publication of application: 23.12.2020
(73) Proprietor: NIO (ANHUI) HOLDING CO., LTD, Economic and Technological Development Zone Hefei Anhui 230601 (CN)
(72) Inventor: HAN, Fang, Shanghai 201804 (CN)
(74) Representative: Bjerkén Hynell KB
(86) International application number: PCT/CN2019/074435
(87) International publication number: WO 2019/154328

(56) References cited:
- EP-A1- 1 561 618
- WO-A1-2016/179167
- CN-A- 106 608 158
- CN-A- 107 310 350
- CN-A- 107 310 350
- CN-U- 205 573 552
- DE-A1-102007 050 321
- DE-B4-102016 000 666
- FR-B1- 2 868 017
- KR-A- 20150 139 228
- US-B1- 8 196 902

## Description

### Technical Field

The invention relates to a fragrance assembly, and in particular to an on-vehicle fragrance assembly.

### Background Art

A fragrance system on a vehicle may be configured to adjust the air inside the vehicle to make a driver and a passenger in the vehicle feel more comfortable. Existing vehicle fragrance systems are all mounted after the production of finished vehicles is completed, and the fragrance system is not integrated with the finished vehicle, resulting in the fragrance system and the shape of the finished vehicle being not unified; and meanwhile, there are also problems that the fragrance cannot operate independently from an air conditioning system, the smell is single, the concentration cannot be adjusted, and the placement is unsafe due to the unstable fixation of a fragrance device, such that diversified individual demands of users cannot be met.

CN107310350A discloses a vehicle-mounted air conditioner. The vehicle-mounted air conditioner comprises a controller, air purification actuator and a fragrance actuator, wherein the controller is configured to control the operation of the air purification actuator and the operation of the fragrance actuator; the air purification actuator is configured to purify air in an automobile; the fragrance actuator is configured to release fragrance into the automobile; and the controller, the air purification actuator and the fragrance actuator are integrated inside the automobile.

KR20150139228A relates to an armrest for a vehicle. The armrest for a vehicle is arranged inside a vehicle, and comprises: an air cleaning unit arranged in the armrest and sucking and filtering air inside a vehicle; an air discharge unit arranged in the armrest and discharging the filtered air to the inside of a vehicle; and a heat exchange module arranged in the air discharge unit.

### Summary of the Invention

The invention for which protection is sought is defined by the independent claim. The dependent claims concern particular embodiments.

The object of the invention is to provide an integrated on-vehicle fragrance assembly to overcome the problems of existing vehicle fragrance systems, and the technical problems to be solved are to alleviate the problems that an existing fragrance system and the shape of a finished vehicle are not unified, the smell is single, and the concentration cannot be adjusted, so that the fragrance system is integrated with the finished vehicle to realise multifunctional linkage at the finished vehicle level, and the smell and concentration of a fragrance of the vehicle can be adjusted to meet individual demands of users.

The following technical solutions are used to realise the object of the invention and solve the technical problems of the invention. The integrated on-vehicle fragrance assembly proposed according to the invention comprises all the features of claim 1.

According to the foregoing integrated on-vehicle fragrance assembly, the fragrance module body comprises a plurality of perfume boxes, perfumes with different flavours are separately placed in all the perfume boxes, each of the perfume boxes is separately provided with an air inlet and an air outlet, and the controller enables, according to the user instruction, an air inlet and an air outlet of a perfume box corresponding to the selected flavour type to be opened or closed.

According to the foregoing integrated on-vehicle fragrance assembly, the fragrance module body comprises a plurality of perfume boxes, perfumes with different flavours are separately placed in all the perfume boxes, each of the perfume boxes is separately provided with an air inlet and an air outlet, and the controller enables, according to the user instruction, an air inlet and an air outlet of a perfume box corresponding to the selected flavour type to be continuously opened.

According to the foregoing integrated on-vehicle fragrance assembly, the fragrance module body comprises a plurality of perfume boxes, perfumes with different flavours are separately placed in all the perfume boxes, each of the perfume boxes is separately provided with an air inlet and an air outlet, and the controller enables, according to the user instruction, an air inlet and an air outlet of a perfume box corresponding to the selected flavour type to be intermittently opened.

According to the foregoing integrated on-vehicle fragrance assembly, the fragrance module body further comprises a housing, wherein the housing is internally provided with a push device, which is controlled by the controller, and all the perfume boxes are provided in the housing; a push rod is separately provided in each of the perfume boxes, an air inlet baffle is fixed at one end of the push rod, and an air outlet baffle is fixed at the other end of the push rod; a push rod hole is separately formed in a position, close to each air outlet, of the housing, and one end, provided with the air outlet baffle, of each push rod separately extends into one push rod hole; restoring devices for restoring all the push rods are provided on the housing; under the control of the controller, the push device pushes the push rod to move from the air inlet to the air outlet so as to open the air inlet and the air outlet of one of the perfume boxes, then the push device stops pushing; and under the action of the restoring device, the push rod is restored, and the air inlet baffle and the air outlet baffle respectively close the air inlet and the air outlet.

According to the foregoing integrated on-vehicle fragrance assembly, the push device is a worm crank mechanism mounted on the housing, wherein the worm crank mechanism is driven by a servo motor, and the servo motor is connected to the controller, which controls the rotation angle of the servo motor.

According to the foregoing integrated on-vehicle fragrance assembly, the restoring devices are a plurality of springs, wherein one end of each of the springs is fixed on the housing, the other end of each of the springs separately abuts against an air outlet baffle of one of the push rods, and each of the push rods separately penetrates the interior of a spring eye of one of the springs.

According to the foregoing integrated on-vehicle fragrance assembly, each of the perfumes with different flavours is separately placed in a perfume bottle, each of the perfume boxes is separately provided with a mounting hole configured to mount the perfume bottle, each of the perfume bottles is separately mounted in the mounting hole of one of the perfume boxes, an outer wall of each of the perfume bottles is separately provided with a clamping device capable of being retracted into the outer wall of the perfume bottle, and the clamping device is clamped on a wall of the mounting hole.

According to the foregoing integrated on-vehicle fragrance assembly, a wind accelerating device is provided at the position, close to all the perfume boxes, in the housing, wherein the wind accelerating device is controlled by the controller and is configured to accelerate the diffusion speed of the flavour.

According to the foregoing integrated on-vehicle fragrance assembly, the wind accelerating apparatus is an air blower mounted on one side close to the air inlets of all the perfume boxes.

According to the foregoing integrated on-vehicle fragrance assembly, the controller is a control chip mounted in the housing.

According to the foregoing integrated on-vehicle fragrance assembly, all the perfume bottles are internally and separately provided with conductive sheets with different resistance values, all the perfume boxes are internally and separately provided with conductive strips electrically connected to the control chip, after all the perfume bottles are placed in all the perfume boxes, each conductive sheet separately makes contact with one conductive strip, and the control chip converts different resistance values into corresponding perfume names to be displayed on a display screen.

By virtue of the above-mentioned technical solutions, the invention at least has the following advantages and beneficial effects:
(1) The integrated on-vehicle fragrance assembly of the invention comprises the controller, the fragrance module body, and the fragrance air channel, wherein the fragrance module body and the fragrance air channel are both provided in the central channel of the vehicle, the controller controls the fragrance module body to diffuse the flavour when needed, the fragrance system is integrated with the finished vehicle so that the fragrance system and the shape of the finished vehicle are unified, and multifunctional linkage at the finished vehicle level is realised.
(2) According to the integrated on-vehicle fragrance assembly of the invention, the fragrance module body comprises the plurality of perfume boxes, the perfumes with different flavours are separately placed in all the perfume boxes, and the controller enables, according to the user instruction, the air inlet and the air outlet of the corresponding perfume box to be opened and then closed, or the air inlet and the air outlet of the corresponding perfume box to be continuously opened, thereby meeting individual demands of different users.
(3) According to the integrated on-vehicle fragrance assembly of the invention, the housing is internally provided with the wind accelerating device to accelerate the diffusion speed of the flavour.
(4) According to the integrated on-vehicle fragrance assembly of the invention, each of the perfumes with different flavours is separately placed in one perfume battle, the outer wall of each perfume bottle is separately provided with the clamping device capable of being retracted into the outer wall of the perfume bottle, and the replacement of the perfume bottles can be realised by means of inserting and extracting, which is easy and convenient.

The above-mentioned description is only an overview of the technical solutions of the invention. In order to more clearly understand the technical means of the invention, the invention can be implemented according to the contents of the description; and in order to make the above-mentioned and other objects, features, and advantages of the invention more obvious and comprehensible, preferred embodiments are particularly given below, and are described in detail as follows with reference to the accompanying drawings.

### Brief Description of the Drawings

Fig. 1 is a schematic structural front view of an integrated on-vehicle fragrance assembly of the invention;
Fig 2 is a schematic structural top view of the integrated on-vehicle fragrance assembly of the invention;
Fig. 3 is a schematic structural diagram of the integrated on-vehicle fragrance assembly, with an air inlet and an air outlet of a perfume box opened, of the invention;
Fig. 4 is a schematic structural diagram of the integrated on-vehicle fragrance assembly, with the air inlet and the air outlet of the perfume box closed, of the invention;
Fig. 5 is a schematic structural diagram of the integrated on-vehicle fragrance assembly, with a perfume bottle taken out, of the invention;
Fig. 6 is a schematic structural diagram of the integrated on-vehicle fragrance assembly, with a perfume bottle mounted, of the invention; and
Fig. 7 is a schematic structural diagram of the perfume bottle of the integrated on-vehicle fragrance assembly of the invention.

**[Description of Symbols]**

| | | | |
|---|---|---|---|
| 10: | Control chip | 20: | Fragrance module body |
| 21: | Perfume box | 22: | Air inlet |
| 23: | Air outlet | 24: | Housing |
| 25: | Push rod | 26: | Air inlet baffle |
| 27: | Air outlet baffle | 28: | Worm crank mechanism |
| 29: | Servo motor | 30: | Fragrance air channel |
| 50: | Air blower | 40: | Spring |
| 60: | Perfume bottle | 61: | Clamping device |
| 62: | Bottle cap | 63: | Metal sheet |

### Detailed Description of Embodiments

In order to further illustrate the technical means used for realising the intended purpose of the invention and the technical effects of the invention, the implementation of an integrated on-vehicle fragrance assembly proposed according to the invention and the features and effects thereof are described in detail hereinafter in conjunction with the accompanying drawings and preferred embodiments.

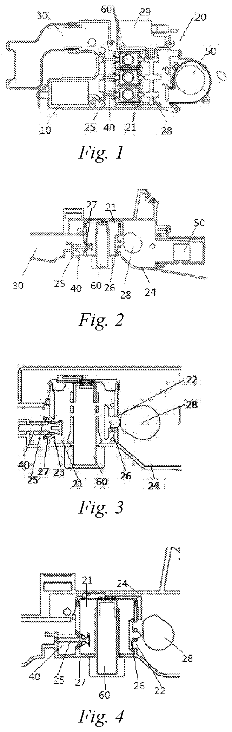

Referring to Fig. 1, an integrated on-vehicle fragrance assembly of the invention comprises a controller, a fragrance module body 20, and a fragrance air channel 30,
wherein the controller is configured to receive a user instruction and control the fragrance module body 20 according to the instruction, and under the control of the controller, the fragrance module body 20 diffuses a flavour when needed; on a finished vehicle production line, the fragrance module body 20 and the fragrance air channel 30 are both mounted in a central channel of a vehicle and are subjected to wire harness docking, the central channel is provided in a central armrest, one end of the fragrance air channel 30 is in communication with the fragrance module body 20, and the other end thereof is in communication with an air outlet of the vehicle; and the air outlet of the vehicle is provided in both sides of a central large screen of an instrument panel, and the flavour diffused by the fragrance module body 20 reaches the air outlet of the vehicle through the fragrance air channel 30, so as to reach the interior of a passenger compartment. A user inputs, through a large screen in the vehicle or a mobile phone, a switch instruction of a fragrance system, and the controller receives the user instruction and controls the fragrance module body 20 according to the user instruction, such that the fragrance module body 20 diffuses the flavour.

According to the invention, the fragrance module body 20 comprises a plurality of perfume boxes 21, perfumes with different flavours are separately placed in all the perfume boxes 21, and each of the perfume boxes 21 is separately provided with an air inlet 22 and an air outlet 23; the user inputs, through the large screen in the vehicle or the mobile phone, the switch instruction of the fragrance system and also selects the flavour type; and the controller enables, according to the user instruction and the selected flavour type, an air inlet and an air outlet of a perfume box 21 containing the above-mentioned flavour type to be opened, such that a flavour of the flavour type selected by the user is diffused from the perfume box, and enters the passenger compartment through the fragrance air channel 30, thereby meeting diversified demands of users. Meanwhile, the user may also select the flavour concentration, when a flavour selected by the user is strong, the controller enables, according to the user instruction, an air inlet and an air outlet of a perfume box 21 containing the flavour type selected by the user to be continuously opened so as to increase the flavour concentration, and when a flavour selected by the user is light, the controller enables, according to a user instruction, an air inlet and an air outlet of a perfume box 21 containing the flavour type selected by the user to be intermittently opened, such that the flavour concentration is light.

In an embodiment, when a main control system of the vehicle detects that a driver is in a fatigue driving state, the main control system of the vehicle automatically selects a fragrance with a refreshing flavour and transmits this instruction to the controller, and the controller controls a perfume box containing a perfume with the refreshing flavour to be opened so as to refresh the user.

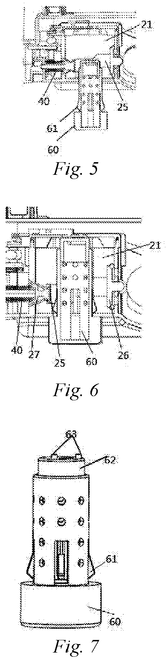

In an embodiment, the fragrance module body 20 further comprises a housing 24, the housing 24 is internally provided with a push device, which is controlled by the controller, and various perfume boxes 21 are provided in the housing 24, as shown in Fig. 1 and Fig. 5. A push rod 25 is separately provided in each of the perfume boxes, an air inlet baffle 26 is fixed at one end of the push rod 25, and an air outlet baffle 27 is fixed at the other end thereof, as shown in Fig. 2 and Fig. 3. A push rod hole is separately formed in a position, close to each air outlet, of the housing 24, and one end, provided with the air outlet baffle 27, of each push rod 25 separately extends into one push rod hole; restoring devices for restoring all the push rods are also provided on the housing 24; the controller controls, according to the user instruction, the push device such that same pushes one push rod 25 to move from the air inlet of the perfume box to the air outlet thereof so as to open the air inlet and the air outlet of one perfume box, then air flow enters the perfume box 21, a flavour selected by the user is diffused from the interior of the perfume box 21, and then the push device stops pushing; and under the action of the restoring device, the push rod 25 is restored, and the air inlet baffle and the air outlet baffle respectively close the air inlet and the air outlet of the perfume box, as shown in Fig. 4. When the user selects another flavour, the controller controls, according to the above-mentioned method, the push device such that same pushes the push rod 25 in the perfume box 21 containing the perfume with the flavour selected by the user. Particularly, as shown in Fig. 1 and Fig. 3, the push device is a worm crank mechanism 28 mounted on the housing 21, the worm crank mechanism 28 is driven by a servo motor 29, the servo motor 29 is connected to the controller, and the controller controls the rotation angle of the servo motor 29 according to the user instruction and the selected flavour type, such that the worm crank mechanism 28 pushes a push rod in a corresponding perfume box 21. For example, when the user selects a rose type flavour, the servo motor 29 leads and drives the worm crank mechanism 28 to rotate by a certain angle, such that the worm crank mechanism 28 pushes a push rod 25 in a perfume box 21 containing a perfume with the rose type flavour, an air inlet and an air outlet of the perfume box 21 containing the perfume with the rose type flavour are opened, and air flow enters the perfume box to bring out the rose type flavour. As an alternative, other devices except for the worm crank mechanism 28 may also be selected as the push device.

In an embodiment, the restoring devices are a plurality of springs 40, as shown in Fig. 1 and Fig. 3, one end of each of the springs 40 is fixed on the housing 24, the other end of each of the springs 24 separately abuts against an air outlet baffle 27 of one push rod 25, and each push rod 25 separately penetrates the interior of a spring eye of one spring 40. In addition, the spring 40 may also be mounted on one end, close to the air inlet, of the housing 24. As an alternative, other devices except for the springs may also be selected as the restoring devices.

In an embodiment, the housing 24 is internally provided with a wind accelerating device, and the wind accelerating device is controlled by the controller to accelerate the diffusion speed of the flavour. Particularly, the wind accelerating apparatus is an air blower 50 mounted on one side close to air inlets of various perfume boxes 21, as shown in Fig. 1 and Fig. 2, the air blower 50 is controlled by the controller, and under the control of the controller, after an air inlet and an air outlet of a certain perfume box 21 are opened, the air blower 50 is turned on to accelerate the diffusion speed of the flavour. As an alternative, an exhaust fan which is mounted on one side, close to air outlets of all the perfume boxes 21, in the housing 24 may also be selected as the wind accelerating apparatus.

According to the invention, each of perfumes with different flavours is separately placed in one perfume bottle 60, as shown in Fig. 5 and Fig. 6, each of the perfume boxes 21 is separately provided with a mounting hole configured to mount a perfume bottle 60, each perfume bottle 60 is separately provided in the mounting hole of one perfume box 21, an outer wall of each perfume bottle 60 is separately provided with a clamping device 61, the clamping device 61 is clamped on a wall of the mounting hole, the clamping device 61 can be retracted into the outer wall of the perfume bottle 60, when the perfume bottle 60 is mounted or dismounted, the clamping device 61 can be retracted into the outer wall of the perfume bottle 60, and the replacement of the perfume bottle can be realised by means of inserting and extracting, which is easy and convenient.

In an embodiment, the controller is a control chip 10 mounted in the housing, and as an alternative, other automatic control device may also be used as the controller.

According to the invention, as shown in Fig. 7, various perfume bottles 60 are internally and separately provided with conductive sheets with different resistance values, various perfume boxes are internally and separately provided with conductive strips electrically connected to the control chip 10, after the perfume bottles 60 are placed in all the perfume boxes 21, each conductive sheet separately makes contact with one conductive strip, and the control chip 10 converts different resistance values into corresponding perfume names to be displayed on a display screen. Particularly, the conductive sheets with different resistance values use metal sheets 63 with different sizes provided in bottle caps 62, the metal sheets 63 may also be provided at other positions of the perfume bottles 60, the conductive sheets with different resistance values may also use other conductive materials with different resistance values, and the conductive strips use conductive metal strips.

The foregoing descriptions are merely preferred embodiments of the invention, and are not intended to limit the invention in any form. Although the invention has been disclosed as above by means of the preferred embodiments, these embodiments are not for defining the invention. Those skilled in the art can make certain alterations or modifications by using the technical contents disclosed above without departing from the scope of the invention as defined by the claims so as to arrive at equivalent embodiments with equivalent changes.

However, any simple amendments, equivalent changes, and modifications made to the above embodiments according to the claims of the invention without departing from the contents of the claims of the invention still fall within the scope of the claims of the invention.

## Claims

1. An integrated on-vehicle fragrance assembly, comprising
a fragrance module body (20), wherein the fragrance module body (20) is provided in a central channel of a vehicle, and the fragrance module body (20) is controlled by a controller to diffuse a flavour when needed, the fragrance module body (20) comprises a plurality of perfume boxes (21), perfumes with different flavours are separately placed in all the perfume boxes (21), each of the perfume boxes (21) is separately provided with an air inlet (22) and an air outlet (23);
a fragrance air channel (30), wherein the fragrance air channel (30) is provided in the central channel of the vehicle, one end of the fragrance air channel (30) is in communication with the fragrance module body (20), and the other end thereof is in communication with an air outlet of the vehicle; and
the controller, wherein the controller is configured to receive a user instruction and control the fragrance module body (20) according to the instruction,
**characterized in that:** each of the perfumes with different flavours is separately placed in a perfume bottle (60), each of the perfume boxes (21) is separately provided with a mounting hole configured to mount the perfume bottle (60), each of the perfume bottles (60) is separately mounted in the mounting hole of one of the perfume boxes (21), an outer wall of each of the perfume bottles (60) is separately provided with a clamping device (61) capable of being retracted into the outer wall of the perfume bottle (60), and the clamping device (61) is clamped on a wall of the mounting hole, and
all the perfume bottles (60) are internally and separately provided with conductive sheets with different resistance values, all the perfume boxes are internally and separately provided with conductive strips electrically connected to the control chip, after all the perfume bottles (60) are placed in all the perfume boxes (21), each conductive sheet separately makes contact with one conductive strip, and the control chip converts different resistance values into corresponding perfume names to be displayed on a display screen.

2. The integrated on-vehicle fragrance assembly according to claim 1, **characterized in that:**
the controller enables, according to the user instruction, the air inlet (22) and the air outlet (23) of the perfume box (21) corresponding to the selected flavour type to be opened or closed.

3. The integrated on-vehicle fragrance assembly according to claim 1, **characterized in that:**
the controller enables, according to the user instruction, the air inlet (22) and the air outlet (23) of the perfume box (21) corresponding to the selected flavour type to be continuously opened.

4. The integrated on-vehicle fragrance assembly according to claim 1, **characterized in that:**
the controller enables, according to the user instruction, the air inlet (22) and the air outlet (23) of the perfume box (21) corresponding to the selected flavour type to be intermittently opened.

5. The integrated on-vehicle fragrance assembly according to claim 2, **characterized in that:** the fragrance module body (20) further comprises a housing (24), wherein the housing (24) is internally provided with a push device, which is controlled by the controller, and all the perfume boxes (21) are provided in the housing (24); a push rod (25) is separately provided in each of the perfume boxes (21), an air inlet baffle (26) is fixed at one end of the push rod (25), and an air outlet baffle (27) is fixed at the other end of the push rod (25); a push rod hole is separately formed in a position, close to each air outlet (23), of the housing (24), and one end, provided with the air outlet baffle (27), of each push rod (25) separately extends into one push rod hole; restoring devices for restoring all the push rods (25) are provided on the housing (24); under the control of the controller, the push device pushes the push rod (25) to move from the air inlet (22) to the air outlet (23) so as to open the air inlet (22) and the air outlet (23) of one of the perfume boxes (21), then the push device stops pushing; and under the action of the restoring device, the push rod is restored, and the air inlet baffle (26) and the air outlet baffle (27) respectively close the air inlet (22) and the air outlet (23).

6. The integrated on-vehicle fragrance assembly according to claim 5, **characterized in that:**
the push device is a worm crank mechanism mounted on the housing (24), wherein the worm crank mechanism is driven by a servo motor, and the servo motor is connected to the controller, which controls the rotation angle of the servo motor.

7. The integrated on-vehicle fragrance assembly according to claim 5, **characterized in that:**
the restoring devices are a plurality of springs, one end of each of the springs is fixed on the housing (24), the other end of each of the springs separately abuts against an air outlet baffle (27) of one of the push rods (25), and each of the push rods (25) separately penetrates the interior of a spring eye of one of the springs.

8. The integrated on-vehicle fragrance assembly according to claim 2, **characterized in that:**
a wind accelerating device is provided at the position, close to all the perfume boxes (21), in the housing, wherein the wind accelerating device is controlled by the controller and is configured to accelerate the diffusion speed of the flavour.

9. The integrated on-vehicle fragrance assembly according to claim 8, **characterized in that:**
the wind accelerating apparatus is an air blower mounted on one side close to the air inlets (22) of all the perfume boxes (21).

10. The integrated on-vehicle fragrance assembly according to claim 1, **characterized in that:**
the controller is a control chip (10) mounted in the housing (24).

## Patentansprüche

1. Integrierte, fahrzeuginterne Duftanordnung, umfassend
einen Duftmodulkörper (20), wobei der Duftmodulkörper (20) in einem mittleren Kanal eines Fahrzeugs bereitgestellt ist und der Duftmodulkörper (20) durch eine Steuerung gesteuert wird, um bei Bedarf ein Aroma zu zerstäuben, wobei der Duftmodulkörper (20) eine Mehrzahl von Parfümbehältnissen (21) umfasst, wobei Parfüme mit unterschiedlichen Aromen separat in allen Parfümbehältnissen (21) platziert sind, wobei jedes der Parfümbehältnisse (21) separat mit einem Lufteinlass (22) und einem Luftauslass (23) versehen ist;
einen Duftluftkanal (30), wobei der Duftluftkanal (30) in dem mittleren Kanal des Fahrzeugs bereitgestellt ist, wobei ein Ende des Duftluftkanals (30) mit dem Duftmodulkörper (20) in Verbindung steht und das andere Ende davon mit einem Luftauslass des Fahrzeugs in Verbindung steht; und
die Steuerung, wobei die Steuerung dazu ausgelegt ist, eine Benutzeranweisung zu empfangen und den Duftmodulkörper (20) gemäß der Anweisung zu steuern,
**dadurch gekennzeichnet, dass**: jedes der Parfüme mit unterschiedlichen Aromen separat in einer Parfümflasche (60) platziert ist, jedes der Parfümbehältnisse (21) separat mit einem Montageloch versehen ist, das dazu ausgelegt ist, die Parfümflasche (60) zu montieren, jede der Parfümflaschen (60) separat in dem Montageloch eines der Parfümbehältnisse (21) montiert ist, eine Außenwand jeder der Parfümflaschen (60) separat mit einer Klemmvorrichtung (61) versehen ist, die in die Außenwand der Parfümflasche (60) zurückgezogen werden kann, und die Klemmvorrichtung (61) an eine Wand des Montagelochs geklemmt ist, und
wobei alle Parfümflaschen (60) innen und separat mit leitfähigen Folien mit unterschiedlichen Widerstandswerten versehen sind, alle Parfümbehältnisse innen und separat mit leitfähigen Streifen versehen sind, die elektrisch mit dem Steuerchip verbunden sind, nachdem alle Parfümflaschen (60) in allen Parfümbehältnissen (21) platziert sind, jede leitfähige Folie separat einen Kontakt mit einem der leitfähigen Streifen herstellt, und der Steuerchip unterschiedliche Widerstandswerte in entsprechende auf einem Anzeigebildschirm anzuzeigende Parfümnamen umwandelt.

2. Integrierte, fahrzeuginterne Duftanordnung nach Anspruch 1, **dadurch gekennzeichnet, dass**:
die Steuerung ermöglicht, dass gemäß der Benutzeranweisung der Lufteinlass (22) und der Luftauslass (23) des Parfümbehältnisses (21), das der ausgewählten Aromaart entspricht, geöffnet und geschlossen werden.

3. Integrierte, fahrzeuginterne Duftanordnung nach Anspruch 1, **dadurch gekennzeichnet, dass**:
die Steuerung ermöglicht, dass gemäß der Benutzeranweisung der Lufteinlass (22) und der Luftauslass (23) des Parfümbehältnisses (21), das der ausgewählten Aromaart entspricht, dauerhaft geöffnet werden.

4. Integrierte, fahrzeuginterne Duftanordnung nach Anspruch 1, **dadurch gekennzeichnet, dass**:
die Steuerung ermöglicht, dass gemäß der Benutzeranweisung der Lufteinlass (22) und der Luftauslass (23) des Parfümbehältnisses (21), das der ausgewählten Aromaart entspricht, intermittierend geöffnet werden.

5. Integrierte, fahrzeuginterne Duftanordnung nach Anspruch 2, **dadurch gekennzeichnet, dass**: der Duftmodulkörper (20) ferner ein Gehäuse (24) umfasst, wobei das Gehäuse (24) innen mit einer Druckvorrichtung versehen ist, die durch die Steuerung gesteuert wird, und alle Parfümbehältnisse (21) in dem Gehäuse (24) bereitgestellt sind; eine Druckstange (25) separat in jedem der Parfümbehältnisse (21) bereitgestellt ist, wobei ein Lufteinlassleitblech (26) an einem Ende der Druckstange (25) fixiert ist und ein Luftauslassleitblech (27) an dem anderen Ende der Druckstange (25) fixiert ist; ein Druckstangenloch separat in einer Position nahe zu jedem Luftauslass (23) des Gehäuses (24) ausgebildet ist und sich ein mit dem Luftauslassleitblech (27) versehenes Ende jeder Druckstange (25) separat in ein Druckstangenloch erstreckt; Rückstellvorrichtungen zum Rückstellen aller Druckstangen (25) an dem Gehäuse (24) bereitgestellt sind; unter der Steuerung der Steuerung die Druckvorrichtung so auf die Druckstange (25) drückt, dass sie sich von dem Lufteinlass (22) zu dem Luftauslass (23) bewegt, um den Lufteinlass (22) und den Luftauslass (23) eines der Parfümbehältnisse (21) zu öffnen, wobei dann die Druckvorrichtung aufhört, zu drücken; und unter der Wirkung der Rückstellvorrichtung die Druckstange rückgestellt wird und das Lufteinlassleitblech (26) und das Luftauslassleitblech (27) den Lufteinlass (22) bzw. den Luftauslass (23) schließen.

6. Integrierte, fahrzeuginterne Duftanordnung nach Anspruch 5, **dadurch gekennzeichnet, dass**:
die Druckvorrichtung ein an dem Gehäuse (24) montierter Schneckenkurbelmechanismus ist, wobei der Schneckenkurbelmechanismus durch einen Servomotor angetrieben wird und der Servomotor mit der Steuerung verbunden ist, die den Drehwinkel des Servomotors steuert.

7. Integrierte, fahrzeuginterne Duftanordnung nach Anspruch 5, **dadurch gekennzeichnet, dass**:
die Rückstellvorrichtungen eine Mehrzahl von Federn sind, wobei ein Ende jeder der Federn an dem Gehäuse (24) fixiert ist, das andere Ende jeder der Federn separat an einem Luftauslassleitblech (27) einer der Druckstangen (25) anliegt und jede der Druckstangen (25) separat durch das Innere eines Federauges einer der Federn dringt.

8. Integrierte, fahrzeuginterne Duftanordnung nach Anspruch 2, **dadurch gekennzeichnet, dass**:
eine Windbeschleunigungseinrichtung an der Position nahe zu allen Parfümbehältnissen (21) in dem Gehäuse bereitgestellt ist, wobei die Windbeschleunigungseinrichtung durch die Steuerung gesteuert wird und dazu ausgelegt ist, die Zerstäubungsgeschwindigkeit des Aromas zu beschleunigen.

9. Integrierte, fahrzeuginterne Duftanordnung nach Anspruch 8, **dadurch gekennzeichnet, dass**:
die Windbeschleunigungseinrichtung ein Luftgebläse ist, das an einer Seite nahe zu den Lufteinlässen (22) aller Parfümbehältnisse (21) montiert ist.

10. Integrierte, fahrzeuginterne Duftanordnung nach Anspruch 1, **dadurch gekennzeichnet, dass**:
die Steuerung ein Steuerchip (10) ist, der in dem Gehäuse (24) montiert ist.

## Revendications

1. Ensemble parfumé intégré dans un véhicule, comprenant
un corps de module de parfum (20), dans lequel le corps de module de parfum (20) est placé dans un canal central d'un véhicule, et le corps de module de parfum (20) est commandé par un dispositif de commande pour diffuser un arôme si nécessaire, le corps de module de parfum (20) comprend une pluralité de boîtes de parfum (21), des parfums ayant différents arômes sont placés séparément dans toutes les boîtes de parfum (21), chacune des boîtes de parfum (21) est munie séparément d'une entrée d'air (22) et d'une sortie d'air (23) ;
un canal d'air parfumé (30), dans lequel le canal d'air parfumé (30) est placé dans le canal central du véhicule, une extrémité du canal d'air parfumé (30) est en communication avec le corps de module de parfum (20), et son autre extrémité est en communication avec une sortie d'air du véhicule ; et
le dispositif de commande, dans lequel le dispositif de commande est configuré pour recevoir une instruction d'utilisateur et commander le corps de module de parfum (20) en fonction de l'instruction,
**caractérisé en ce que** : chacun des parfums ayant différents arômes est placé séparément dans une bouteille de parfum (60), chacune des boîtes de parfum (21) est munie séparément d'un trou de montage conçu pour monter la bouteille de parfum (60), chacune des bouteilles de parfum (60) est montée séparément dans le trou de montage de l'une des boîtes de parfum (21), une paroi externe de chacune des bouteilles de parfum (60) est munie séparément d'un dispositif de serrage (61) pouvant être rétracté dans la paroi externe de la bouteille de parfum (60), et le dispositif de serrage (61) est serré sur une paroi du trou de montage, et
toutes les bouteilles de parfum (60) sont munies séparément à l'intérieur de feuilles conductrices de différentes valeurs de résistance, toutes les boîtes de parfum sont munies séparément à l'intérieur de bandes conductrices électriquement connectées à la puce de commande, après que toutes les bouteilles de parfum (60) sont placées dans toutes les boîtes de parfum (21), chaque feuille conductrice entre en contact séparément avec une bande conductrice, et la puce de commande convertit différentes valeurs de résistance en noms de parfum correspondants à afficher sur un écran d'affichage.

2. Ensemble parfumé intégré dans un véhicule selon la revendication 1, **caractérisé en ce que** :
le dispositif de commande permet d'ouvrir ou de fermer, en fonction de l'instruction d'utilisateur, l'entrée d'air (22) et la sortie d'air (23) de la boîte de parfum (21) correspondant au type d'arôme sélectionné.

3. Ensemble parfumé intégré dans un véhicule selon la revendication 1, **caractérisé en ce que** :
le dispositif de commande permet d'ouvrir en continu, en fonction de l'instruction d'utilisateur, l'entrée d'air (22) et la sortie d'air (23) de la boîte de parfum (21) correspondant au type d'arôme sélectionné.

4. Ensemble parfumé intégré dans un véhicule selon la revendication 1, **caractérisé en ce que** :
le dispositif de commande permet d'ouvrir de façon intermittente, en fonction de l'instruction d'utilisateur, l'entrée d'air (22) et la sortie d'air (23) de la boîte de parfum (21) correspondant au type d'arôme sélectionné.

5. Ensemble parfumé intégré dans un véhicule selon la revendication 2, **caractérisé en ce que** : le corps de module de parfum (20) comprend en outre un logement (24), dans lequel le logement (24) est muni à l'intérieur d'un dispositif de poussée, qui est commandé par le dispositif de commande, et toutes les boîtes de parfum (21) sont placées dans le logement (24) ; une tige de poussée (25) est placée séparément dans chacune des boîtes de parfum (21), un déflecteur d'entrée d'air (26) est fixé à une extrémité de la tige de poussée (25), et un déflecteur de sortie d'air (27) est fixé à l'autre extrémité de la tige de poussée (25) ; un trou de tige de poussée est formé séparément à une position, proche de chaque sortie d'air (23), du logement (24), et une extrémité, munie du déflecteur de sortie d'air (27), de chaque tige de poussée (25) s'étend séparément dans un trou de tige de poussée ; des dispositifs de restauration destinés à restaurer toutes les tiges de poussée (25) sont placés dans le logement (24) ; sous la commande du dispositif de commande, le dispositif de poussée pousse la tige de poussée (25) en déplacement de l'entrée d'air (22) à la sortie d'air (23) de façon à ouvrir l'entrée d'air (22) et la sortie d'air (23) de l'une des boîtes de parfum (21), puis le dispositif de poussée cesse de pousser ; et sous l'action du dispositif de restauration, la tige de poussée est restaurée, et le déflecteur d'entrée d'air (26) et le déflecteur de sortie d'air (27) ferment respectivement l'entrée d'air (22) et la sortie d'air (23).

6. Ensemble parfumé intégré dans un véhicule selon la revendication 5, **caractérisé en ce que** :
le dispositif de poussée est un mécanisme à manivelle sans fin monté sur le logement (24), dans lequel le mécanisme à manivelle sans fin est entraîné par un servomoteur, et le servomoteur est connecté au dispositif de commande, qui commande l'angle de rotation du servomoteur.

7. Ensemble parfumé intégré dans un véhicule selon la revendication 5, **caractérisé en ce que** :
les dispositifs de restauration sont une pluralité de ressorts, une extrémité de chacun des ressorts est fixée sur le logement (24), l'autre extrémité de chacun des ressorts vient en butée séparément contre un déflecteur de sortie d'air (27) de l'une des tiges de poussée (25), et chacune des tiges de poussée (25) pénètre séparément à l'intérieur d'un œil de ressort de l'un des ressorts.

8. Ensemble parfumé intégré dans un véhicule selon la revendication 2, **caractérisé en ce que** :
un dispositif d'accélération de vent est placé à une position, proche de toutes les boîtes de parfum (21), dans le logement, dans lequel le dispositif d'accélération de vent est commandé par le dispositif de commande et est conçu pour accélérer la vitesse de diffusion de l'arôme.

9. Ensemble parfumé intégré dans un véhicule selon la revendication 8, **caractérisé en ce que** :
l'appareil d'accélération de vent est un souffleur d'air monté sur un côté proche des entrées d'air (22) de toutes les boîtes de parfum (21).

10. Ensemble parfumé intégré dans un véhicule selon la revendication 1, **caractérisé en ce que** :
le dispositif de commande est une puce de commande (10) montée dans le logement (24).
